# EUROPEAN PATENT SPECIFICATION

(11) **EP 3 982 823 B1**
(45) Date of publication and mention of the grant of the patent: **02.12.2026**
(21) Application number: 20823537.4
(22) Date of filing: 08.06.2020
(51) Int. Cl.: A61B 5/00, A61B 5/20, A61M 27/00, A61B 5/026, A61B 5/103, A61F 5/44, A61M 25/00, A61F 2/00

(54) **AUTOMATED CATHETER**
AUTOMATISCHE KATHETER
CATHÉTER AUTOMATISÉ

(30) Priority: 13.06.2019 US 201962861165 P; 13.06.2019 US 201962861169 P
(43) Date of publication of application: 20.04.2022
(73) Proprietor: Tessefi Innovations, Inc., Rocky Mount, NC 27804 (US)
(72) Inventor: KEELEN, Mengesha, Tucson, Arizona 85711 (US)
(74) Representative: Maiwald GmbH
(86) International application number: PCT/US2020/036640
(87) International publication number: WO 2020/251893

(56) References cited:
- WO-A1-2017/070117
- WO-A1-2018/180229
- KR-A- 20030 069 757
- US-A1- 2005 256 447
- US-A1- 2013 165 877
- US-A1- 2016 051 176
- US-A1- 2016 310 711
- US-A1- 2017 113 000
- US-A1- 2017 136 209
- US-A1- 2019 069 831
- US-B2- 7 264 616

## Description

### CROSS-REFERENCE TO RELATED APPLICATIONS

The present application claims priority to U.S. Provisional Patent Application Serial No. 62/861,169, filed June 13, 2019, and U.S. Provisional Patent Application Serial No. 62/861,165, filed June 13, 2019.

### TECHNICAL FIELD

The subject matter herein generally relates to the field of catheters and related devices. The subject matter herein more particularly relates to, automated catheter devices and related systems, including urinary catheters.

### BACKGROUND

Modern medical catheters, particularly Foley catheters, are used to collect urine in patients while admitted to hospitals and medical facilities. However, the amount of urine collected by the catheter is not automatically calculated, and the data is not available electronically via a computer. Instead, urine output must be manually measured or observed, and the data manually charted or recorded when time permits. This can be detrimental to the patient because if a nurse, Nursing Assistant, technician or other medical professional forgets to chart the amount of periodic output a provider will assume there was no urine output. There are numerous potentials for errors including, but not limited to spilled urine and incorrect or imprecise measurements. This is crucial in providing proper medical care. For example, if a patient is admitted with a diagnosis such as diabetes insipidus, proper recording and utilization of urine output can be a life or death proposition.

In a similar vein, chest tubes are used to collect air, fluid, pleural effusion, blood, chyle, or pus from the intrathoracic space of a patient while admitted to hospitals and medical facilities, and/or otherwise under medical care. However, the amount of air, fluid, pleural effusion, blood, chyle, or pus collected by the chest tube is not automatically calculated, and the data is not available electronically via a computer. Instead, the amount of air or fluid output must be manually measured or observed, and the data manually charted or recorded when time permits. Similarly to the urine catheter issues described above, if the nurse, or other professional, fails to chart the periodic air, fluid, pleural effusion, blood, chyle, or pus collected from the patient, a provider will assume there was no output. These types of charting are critical for receiving the proper treatment and, just like with the catheter, the potential consequence for error could be deadly. For example, if a patient is admitted with a particular condition, proper recording and utilization of air, fluid, pleural effusion, blood, chyle, or pus output can be a life or death proposition.

Thus, what is needed is an automated system delivering results in real-time or a device that collects, measures and/or records urine output and/or air, flud, pleural effusion, blood, chyle, and/or puss output with minimal or no effort by medical personnel. Such need is addressed by the devices and system described herein.

Documents WO2918/180229A1 and WO 2017/070117 A1 both disclose devices for draining fluids, such as urine from a subject.

### SUMMARY

The invention is defined by the appended claims.

Any aspect and subject matter related to chest tubes do not form part of the claimed invention.

In accordance with this disclosure, smart catheter and smart chest tube devices and systems are provided. In one aspect, a smart catheter is provided, the smart catheter comprising: a flexible tube with an opening at a first end; a drainage port at an opposing second end; an inflatable balloon at the first end, and proximate to the opening; a balloon port at the second end and proximate to the drainage port; and a measuring device, comprising one or more sensors, integrated into or attached to the flexible tube and configured to measure an amount of fluid flowing therethrough. In some embodiments, the smart catheter is configured to drain urine from a bladder of a patient, and the measuring device is a flow meter sensor. In some embodiments, the drainage port is configured to connect or otherwise attach to one or more collection reservoirs; and the smart catheter is configured to deposit urine in the one or more collection reservoirs.

In some further embodiments, the flow meter sensor is configured to measure the amount of urine deposited into the one or more collection reservoirs. In some embodiments, the flexible tube comprises two separated channels or lumens running along a length of the flexible tube, wherein a first lumen, open at both ends, connects the opening at the first end to the drainage port at the second end, wherein the second lumen connects the inflatable balloon to the balloon port. In some embodiments, the measuring device is integrated into and/or along a length of the flexible tube at any suitable location. In some embodiments, the measuring device comprises a transmitter that is configured to transmit data to one or more processors in communication with the measuring device.

In some further embodiments, the transmitted data comprises urine flow data; the one or more processors is configured to store and monitor the urine flow data; the one or more processors is configured to monitor an amount of urine deposited into one or more collection reservoirs of the smart catheter; and the one or more processors is configured to transmit a warning signal or trigger an alarm when at least one of the one or more collection reservoirs of the smart catheter reaches one or more thresholds. In some embodiments, the one or more processors is configured to transmit a warning signal or trigger an alarm when there is a malfunction of the smart catheter. In some embodiments, components of the smart catheter are housed in an enclosure comprising a display screen; and the display screen is configured to display fill levels of one or more collection reservoirs of the smart catheter.

In some embodiments, the enclosure further comprises a transparent or non-transparent door; and wherein each of the one or more collection reservoirs is either transparent or non-transparent. In some embodiments, one or more of the collection reservoirs comprises a total dissolved solids meter and/or a color sensor configured to detect and measure a color or shade of a fluid. In some embodiments, one or more of the collection reservoirs comprises a quick connect port to connect the one or more collection reservoirs to the smart catheter. In some embodiments, when a first collection reservoir is removed from the smart catheter, the smart catheter is configured to automatically select a second collection reservoir and start draining urine into the second collection reservoir. In some embodiments, each of the one or more collection reservoirs comprises an electrical connection configured to provide power to various components in a respective collection reservoir and to connect the one or more processors to the various components in the respective collection reservoir.

In another aspect, a smart catheter system is provided, the smart catheter system comprising a smart catheter of any of the above embodiments; and an external device, wherein the external device comprises a tablet, computer, phone, smart watch, audio device or display device. In some embodiments, wherein the system further comprises: a power source, one or more processors, memory, a receiver or transmitter, a display, an accelerometer, a speaker and/or a tactile signal device, wherein the smart catheter, power source, one or more processors, memory, receiver or transmitter, display, accelerometer, speaker or tactile signal device are interconnected with one another. In some embodiments, the display is configured to display information about the patient and/or the smart catheter.

In some embodiments, the display is configured to display identification information about the patient as well as information regarding the patient's urine; and wherein the display is configured to display information about a capacity of each of the one or more collection reservoirs or a warning or error message. In some embodiments, the smart catheter system comprises a computer program product comprising computer executable instructions embodied in a computer readable medium for performing steps comprising receiving an electrical signal from a measuring apparatus, processing the electrical signal to calculate data pertaining to a measured volume, and relaying the data to the electronic display, speaker, tactile signal device or external device. In some embodiments, the smart catheter system are configured to measure and calculate a volume of urine or other body fluid produced by a patient every 30 minutes or Q one hour, wherein the calculated volume is processed by a computer and entered into an electronic charting system.

In some embodiments, the smart catheter system is configured to measure and calculate the volume of urine or other body fluid produced by a patient and activate an alarm if the volume of urine and/or other body fluid is above and/or below a predetermined threshold. In some embodiments, the smart catheter or smart catheter system of any of the above embodiments, where the smart catheter or smart catheter system is configured as a chest tube.

In another aspect a smart chest tube is provided, the smart chest tube comprising: a flexible tube with an opening at a first end; a drainage port at the opposing second end; and a measuring device, comprising one or more sensors, integrated into or attached to the flexible tube and configured to measure an amount of fluid flowing therethrough. In some embodiments, the chest tube is configured to drain air, fluid, pleural effusion, blood, chyle, or pus from a chest cavity of a patient, and wherein the measuring device is a fluid flow meter sensor. In some embodiments, the drainage port is configured to connect or otherwise attach to one or more collection reservoirs; and the smart chest tube is configured to deposit air, fluid, pleural effusion, blood, chyle, or pus in the one or more collection reservoirs. In some embodiments, the flow meter sensor is configured to measure the amount of air, fluid, pleural effusion, blood, chyle, or pus deposited into the one or more collection reservoirs.

In some further embodiments, the measuring device is integrated into and/or along a length of the flexible tube at any suitable location. In some embodiments, the measuring device comprises a transmitter that is configured to transmit data to one or more processors in communication with the measuring device. In some embodiments, the transmitted data comprises flow data regarding air, fluid, pleural effusion, blood, chyle, or pus flowing through the flexible tube and measured by the measuring device; the one or more processors is configured to store and monitor the flow data; the one or more processors is configured to monitor an amount of air, fluid, pleural effusion, blood, chyle, or pus deposited into one or more collection reservoirs of the smart chest tube; the one or more processors is configured to transmit a warning signal or trigger an alarm when at least one of the one or more collection reservoirs of the smart chest tube reaches one or more thresholds.

In some further embodiments, the one or more processors is configured to transmit a warning signal or trigger an alarm when there is a malfunction of the smart chest tube. In some embodiments, components of the smart chest tube are housed in an enclosure comprising a display screen; and the display screen is configured to display fill levels of one or more collection reservoirs of the smart chest tube. In some embodiments, the chest tube system is configured to measure and calculate a volume of air or a body fluid produced by a patient and activate an alarm if the volume of air or a body fluid is above or below one or more predetermined thresholds.

In some further embodiments, one or more of the collection reservoirs comprises a total dissolved solids meter and/or a color sensor configured to detect and measure a color or shade of a fluid. In some embodiments, one or more of the collection reservoirs comprises a quick connect port to connect the one or more collection reservoirs to the smart chest tube. In some embodiments, when a first collection reservoir is removed from the smart chest tube, the smart chest tube is configured to automatically select a second collection reservoir and start draining body fluid into the second collection reservoir. In some embodiments, each of the one or more collection reservoirs comprises an electrical connection configured to provide power to various components in a respective collection reservoir and to connect the one or more processors to the various components in the respective collection reservoir.

In some embodiments, a smart chest tube system is provided, the system comprising a smart chest tube of any of the above claims; and an external device, wherein the external device comprises a tablet, computer, phone, smart watch, audio device or display device. In some embodiments, the system further comprises: a power source, one or more processors, memory, a receiver or transmitter, a display, an accelerometer, a speaker and/or a tactile signal device, wherein the power source, one or more processors, memory, receiver or transmitter, display, accelerometer, speaker or tactile signal device are interconnected with one another. In some embodiments, the display is configured to display information about the patient and/or the smart catheter.

In some embodiments, the display is configured to display identification information about the patient as well as information regarding the patient's urine; and
wherein the display is configured to display information about a capacity of each of the one or more collection reservoirs or a warning or error message. In some embodiments, the smart chest tube system further comprises a computer program product comprising computer executable instructions embodied in a computer readable medium for performing steps comprising receiving an electrical signal from a measuring apparatus, processing the electrical signal to calculate data pertaining to a measured volume, and relaying the data to the electronic display, speaker, tactile signal device or external device. In some embodiments, the smart chest tube system is configured to measure and calculate a volume of air or a body fluid produced by a patient every 30 minutes or Q one hour, wherein the calculated volume is processed by a computer and entered into an electronic charting system. In some further embodiments, the chest tube system is configured to measure and calculate the volume of air or a body fluid produced by a patient and activate an alarm if the volume of air or a body fluid is above and/or below a predetermined threshold.

Although some of the aspects of the subject matter disclosed herein have been stated hereinabove, and which are achieved in whole or in part by the presently disclosed subject matter, other aspects will become evident as the description proceeds when taken in connection with the accompanying drawings as best described hereinbelow.

### BRIEF DESCRIPTION OF THE DRAWINGS

The features and advantages of the present subject matter will be more readily understood from the following detailed description which should be read in conjunction with the accompanying drawings that are given merely by way of explanatory and non-limiting example, and in which:
FIG. 1A is a perspective view of a smart catheter device according to an embodiment of the presently disclosed subject matter;
FIG. 1B is a perspective view of a smart catheter device according to an embodiment of the presently disclosed subject matter with a display glowing in the dark;
FIG. 2A depicts a front view of a smart catheter device according to an embodiment of the presently disclosed subject matter;
FIG. 2B and FIG. 2C depict a side view of a smart catheter device and illustrates the details of the flexible tube according to an embodiment of the presently disclosed subject matter;
FIG. 3A and FIG. 3B are perspective views of a smart catheter device according to an embodiment of the presently disclosed subject matter with a door of the smart catheter ajar, exposing the internal components of the smart catheter;
FIG. 4A and FIG. 4B are close-up views of vials or collection reservoirs of a smart catheter device according to an embodiment of the presently disclosed subject matter;
FIG. 5 is an exploded view of a vial or collection reservoir of a smart catheter device according to an embodiment of the presently disclosed subject matter;
FIG. 6A is a perspective bottom view and a side view of a smart catheter device according to an embodiment of the presently disclosed subject matter;
FIG. 6B depicts perspective rear views of a smart catheter device according to an embodiment of the presently disclosed subject matter;
FIG. 7 is a close-up perspective top view of a smart catheter device according to an embodiment of the presently disclosed subject matter; and
FIG. 8 is a close-up perspective to view of a smart chest tube device and illustrates the details of the flexible tube according to an embodiment of the presently disclosed subject matter.

### DETAILED DESCRIPTION

The present subject matter provides automated or smart catheter systems and devices and automated or smart chest tube systems and devices. In one aspect, the present subject matter provides smart catheter systems and devices for draining, storing, and measuring urine from a patient and warning or alerting healthcare officials once the urine levels get to a certain level or the device malfunctions. In similar aspect, the present subject matter provides smart chest tube systems and device for draining, storing, and measuring bodily fluids from a patient and warning or alerting healthcare officials once the bodily fluid levels reach a certain threshold. While the following terms are believed to be well understood by one having ordinary skill in the art, the following definitions are set forth to facilitate explanation of the presently disclosed subject matter.

Unless defined otherwise, all technical and scientific terms used herein have the same meaning as commonly understood to one having ordinary skill in the art to which the presently disclosed subject matter belongs. Although, any methods, devices, and materials similar or equivalent to those described herein can be used in the practice or testing of the presently disclosed subject matter, representative methods, devices, and materials are now described.

Following long-standing patent law convention, the terms "a", "an", and "the" refer to "one or more" when used in this application, including the claims. Thus, for example, reference to "a vial" can include a plurality of such vials, and so forth.

Unless otherwise indicated, all numbers expressing quantities of length, diameter, width, and so forth used in the specification and claims are to be understood as being modified in all instances by the terms "about" or "approximately". Accordingly, unless indicated to the contrary, the numerical parameters set forth in this specification and attached claims are approximations that can vary depending upon the desired properties sought to be obtained by the presently disclosed subject matter.

As used herein, the terms "about" and "approximately," when referring to a value or to a length, width, diameter, temperature, time, volume, concentration, percentage, *etc.,* is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate for the disclosed apparatuses and devices.

The term "comprising", which is synonymous with "including" "containing" or "characterized by" is inclusive or open-ended and does not exclude additional, unrecited elements or method steps. "Comprising" is a term of art used in claim language which means that the named elements are essential, but other elements can be added and still form a construct within the scope of the claim.

As used herein, the phrase "consisting of" excludes any element, step, or ingredient not specified in the claim. When the phrase "consists of" appears in a clause of the body of a claim, rather than immediately following the preamble, it limits only the element set forth in that clause; other elements are not excluded from the claim as a whole.

As used herein, the phrase "consisting essentially of" limits the scope of a claim to the specified materials or steps, plus those that do not materially affect the basic and novel characteristic(s) of the claimed subject matter.

With respect to the terms "comprising", "consisting of", and "consisting essentially of", where one of these three terms is used herein, the presently disclosed and claimed subject matter can include the use of either of the other two terms.

As used herein, the term "and/or" when used in the context of a listing of entities, refers to the entities being present singly or in combination. Thus, for example, the phrase "A, B, C, and/or D" includes A, B, C, and D individually, but also includes any and all combinations and sub-combinations of A, B, C, and D.

### Smart Catheter Devices and Systems

Urinary catheters are used to collect urine in patients while admitted to hospitals and medical facilities, as well as at home and for chronic use. The urine output is manually collected and measured by nurses and other medical professionals. Unfortunately, there is no automated system for performing this important task. Instead, urine output must be manually measured or observed, and the data manually charted or recorded, usually hours after. This is a crucial step in providing proper medical care, but due to the manual nature of this task it can easily be overlooked or delayed. An automated or smart system or device that collects, measures and/or records urine output with minimal or no effort by medical personnel would improve efficiency, accuracy, best practice, health care effectiveness and successful patient outcomes. The disclosed devices and systems fill this unmet need. Particularly, the disclosed devices and systems automatically collect, measure, calculate and record urine output and other related data. In some embodiments, such catheters and catheter systems can be referred to as a precise catheter, smart catheter, electronic catheter, and the like, or precise Foley, smart Foley, electronic Foley, and the like.

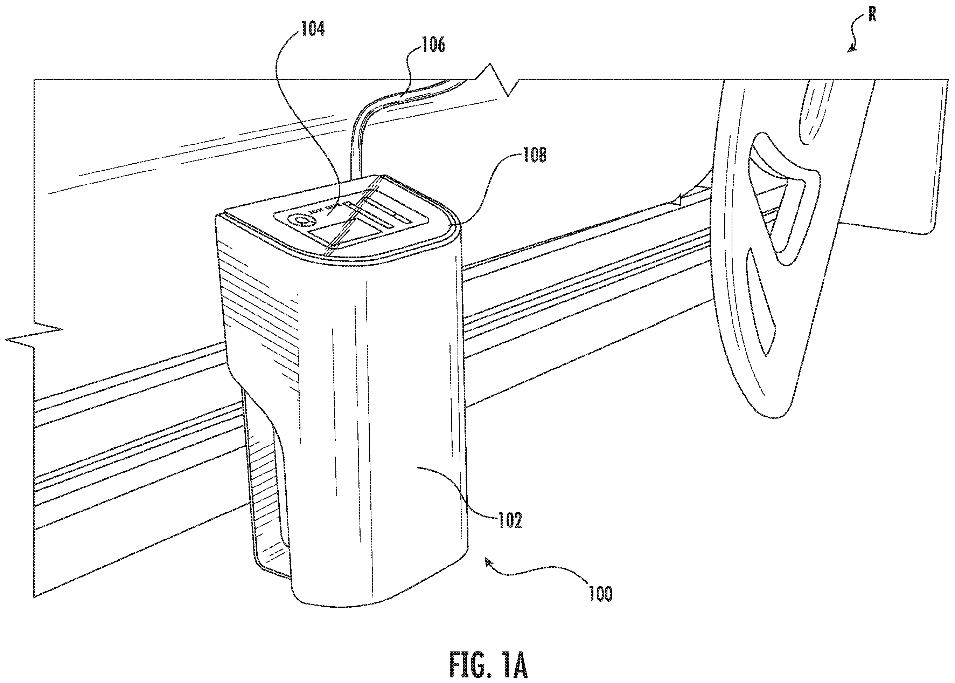

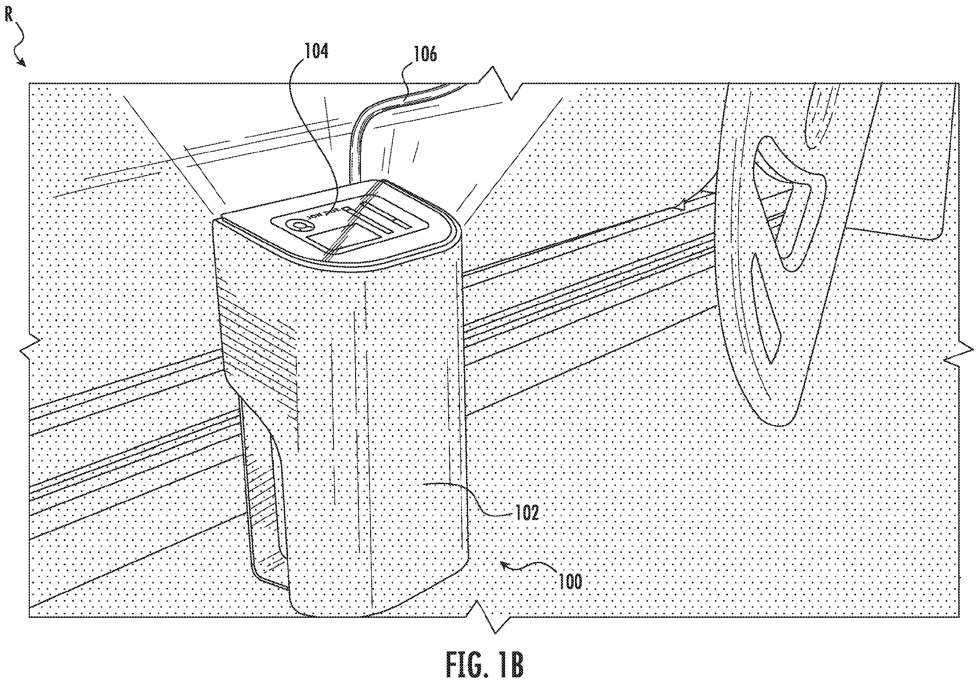

Referring to FIG. 1A, which depicts a perspective view of one embodiment of a possible smart catheter **100** of the present disclosure attached to the side of a hospital bed. In some embodiments, the smart catheter **100** can have an enclosure or housing that houses a subset of its components. The housing or enclosure can have a door **102** that swings open revealing any components within. According to the invention, the automated catheter comprises a flexible tube **106** (i.e., the actual urinary catheter component), and can also comprise a display 104 and a lighting element **108.** Referring to FIG. 1B,
in some embodiments, the lighting element **108** can light up the room it is in like a night light, allowing nurses, doctors, and other health professionals to view the display without disturbing the patient by cutting on the lights in the room to see the contents of the catheter collection reservoir(s). The characteristics of the various components are described further hereinbelow.

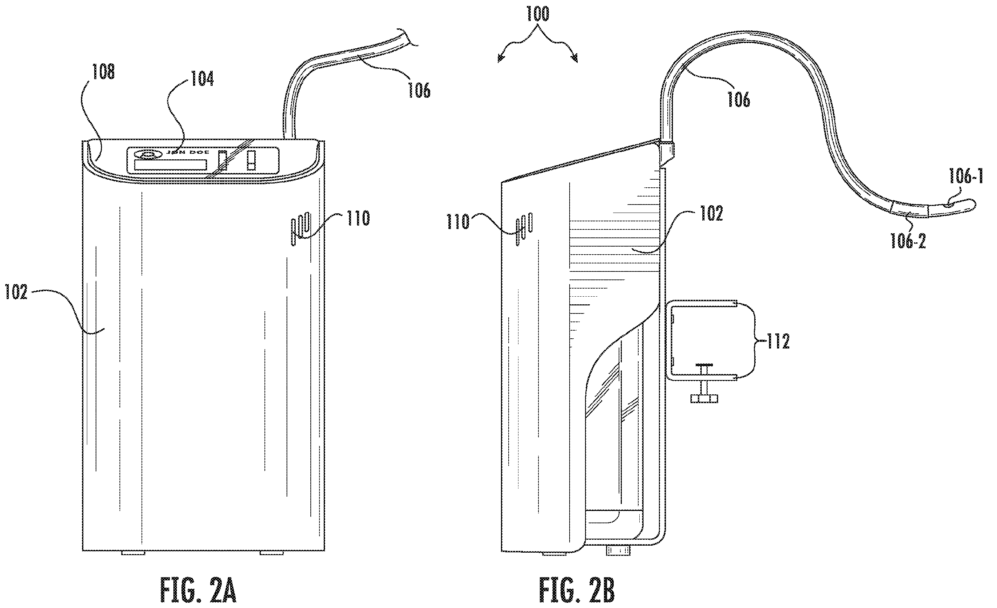

Referring to FIG. 2A, which illustrates a front exterior view of a possible smart catheter **100** of the present disclosure. In some embodiments, the door **102** includes a pattern **110,** grooves, or other indicia indicating the direction in which the door **102** opens. This will allow medical professionals to easily determine which way to open the door to increase efficiencies when trying to inspect or exchange components inside the housing of the smart catheter **100.**

As shown in FIG. 2B, in some embodiments, the smart catheter **100** of the present disclosure can comprise a clamp and/or bracket **112** on the back of the housing, which can be used to temporarily or permanently fasten or attach the smart catheter **100** to a surface, such as, for example, the side of a hospital bed, a table, or other suitable location. In some embodiments, the smart catheter **100** can be configured such that the flexible tube **106** used to drain the urine can ingress into the smart catheter **100** through the top of the housing. In some embodiments, the flexible tube **106** can ingress into the back of the smart catheter **100** or any other suitable location.

According to the invention, the smart catheter **100** of the present disclosure comprises a urinary catheter used to drain urine from the bladder of a patient. In some embodiments, the urinary catheter can comprise, for example and without limitation, a Foley catheter, comprising a flexible tube **106** which a clinician passes through the urethra of a patient and into the patient's bladder to drain urine. In some embodiments, the flexible tube **106** comprises a bladder opening **106-1** at one end and a urine drainage port at an opposing second end (not visible in this view because it is located inside the housing). The bladder opening **106-1** end can be passed through the patient's urethra until the bladder opening **106-1** reaches the patients' bladder. The urine drainage port is configured, according to the invention, to connect or otherwise attach to a collection reservoir, described further herein.

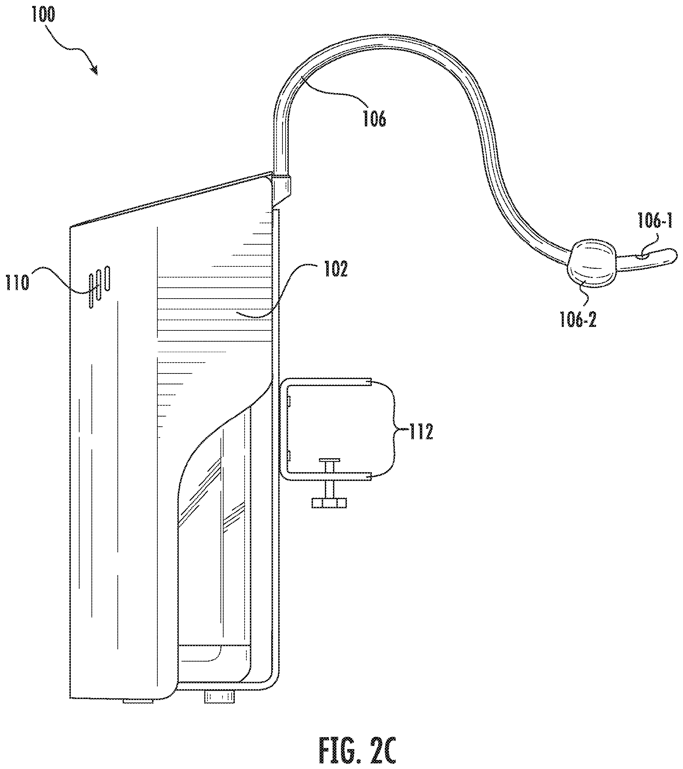

Referring to FIG. 2C, according to the invention, the flexible tube **106** also includes an inflatable balloon **106-2** at or near the end where the bladder opening **106-1** resides, and a balloon port at the opposing end near where the drainage port resides (i.e., connects to the smart catheter housing). In some aspects, the flexible tube **106** has two separate channels or lumens running down its length. One lumen, open at both ends, connects the bladder opening **106-1** to the urine drainage port and is configured to drain urine from the bladder, through the lumen, out the drainage port and into a collection reservoir, such as, for example and without limitation, a collection bag. The other lumen connects the balloon port **106-2** and the balloon. The balloon **106-2** is inflated with sterile water when it lies inside the bladder to stop the catheter or flexible tube **106** from slipping out.

In some embodiments, the flexible tube **106** can be made of silicone or other suitable material and/or coated natural latex. Coatings can including polytetrafluoroethylene, hydrogel, or a silicon elastomer. In some instances, the different properties of these surface coatings can determine the suitable duration of use, e.g. whether the catheter is suitable for 28-day or 3-month indwelling duration. Additionally, catheters are also changed if soiled, leaking, or if an infection is present.

In some embodiments, the smart catheter **100** can comprise one or more processors (not shown), non-transitory computer readable media, and executable instructions used to operate the various automated functions of the device. In some embodiments, the one or more processors can be configured to operate the display **104,** perform various measurements based on sensors described herein, and perform various other functions as described herein.

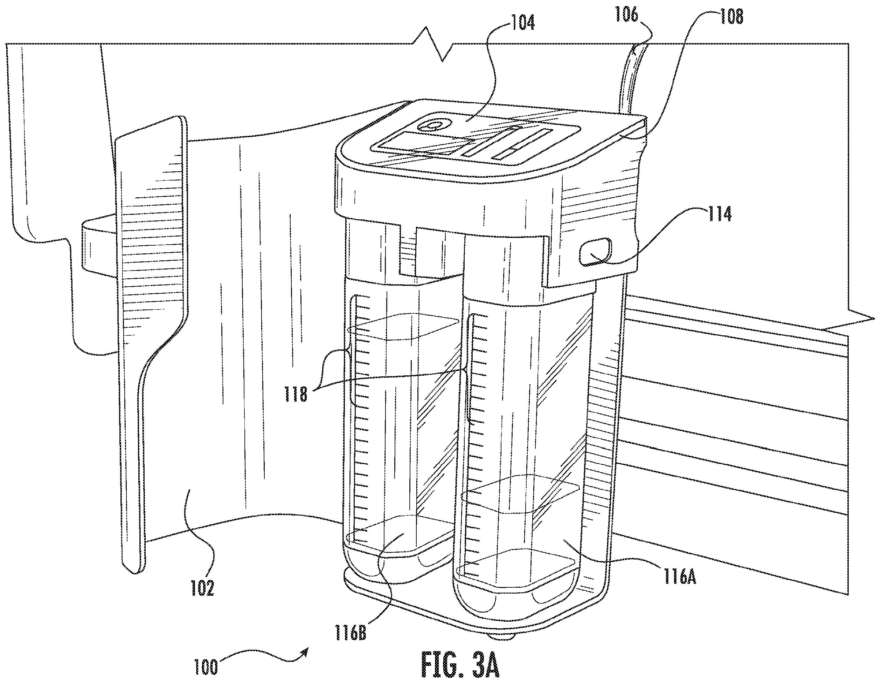

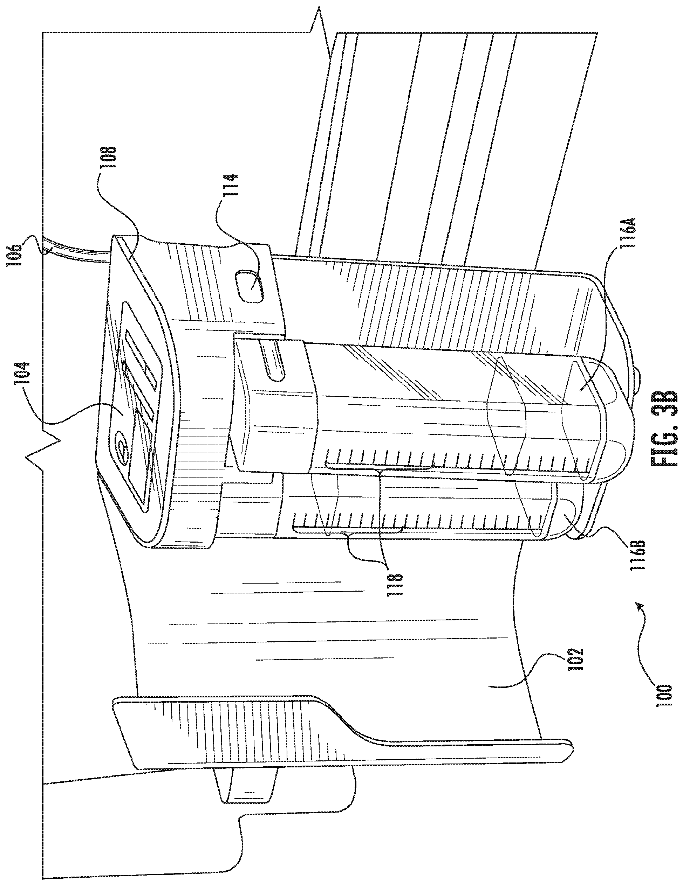

Referring to FIG. 3A and FIG. 3B, in some embodiments, as described above, the housing of the smart catheter **100** can comprise a door **102** configured to open in any suitable direction. For example and without limitation, the door **102** can be hingedly attached to the housing and open up (i.e., like an overhead storage bin in an aircraft) or the door **102** could open out, laterally (i.e., like a typical door). Although the embodiments disclosed in the figures depict a door **102** that is painted, translucent, or non-transparent to obscure the contents of the collection reservoirs **116,** in some embodiments, the door **102** can be clear or transparent so that medical staff can easily visually monitor the contents of the collection reservoirs **116.** According to the invention, the housing of the smart catheter **100** comprises at least two collection reservoirs **116** inside. For example and without limitation, the smart catheter **100** can comprise a first collection reservoir **116A** and a second collection reservoir **116B,** each configured to collect urine or other fluid as shown in FIG. 3A. As depicted in FIG 3A, the first collection reservoir **116A** and the second collection reservoir **116B** can comprise, for example, and without limitation, vials, bags, jars, or any suitable container or reservoir for containing the urine. In some embodiments, each of the first collection reservoir **116A** and the second collection reservoir **116B** can comprise graduated markings **118** on them to help indicate how full they are for a healthcare worker to manually verify their fill levels. In some embodiments, each of the collection reservoirs **116** can be transparent or non-transparent, For example, if medical staff is required to visually monitor the contents of the collection reservoirs **116,** they can be transparent.

As illustrated in FIG. 3B, in some embodiments, the housing of the smart catheter **100** can comprise one or more push buttons **114** to eject one or more of the collection reservoirs. For example and without limitation, in some embodiments, there can be one push button **114** for every collection reservoir or there can be a different push button **114** for each collection reservoir. In some embodiments, the push button **114** and the one or more collection reservoirs **116** can be configured and positioned such that any of the one or more collection reservoirs **116** can be removed using only one hand. For example and without limitation, in some embodiments, the push button **114** can be positioned such that a finger of the hand grasping the body of the collection reservoir **116** can also press the push button **114** to release the collection reservoir **116.** As shown in FIG. 3B, one or more of the collection reservoirs, such as for example first collection reservoir **116A,** is capable of being removed for inspection, emptying, replacement, or any other suitable purpose.

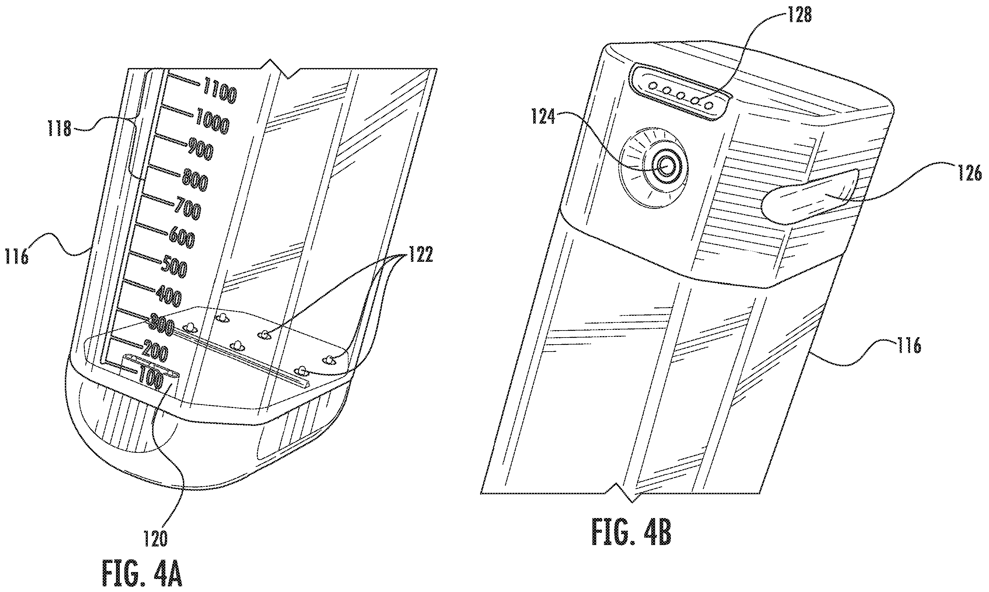

Referring to FIG. 4A and FIG. 4B in some embodiments, the collection reservoir **116** can comprise a total dissolved solids (TDS) meter **120** or sensor configured for measuring the total dissolved solids in the urine. In some embodiments, the TDS meter **120** is in communication with the one or more processors of the smart catheter **100,** wherein the one or more processors is configured to use the TDS meter **120** to capture total dissolved solids measurements of the urine and relay that data or perform various tasks with that data. For example and without limitation, based on outputs from the TDS meter **120,** the one or more processors can be configured to transmit statistics about this data to health provider software, or provide outputs on the display **102.** This information can then be used to ensure that the health of the patient is being monitored.

Additionally, in some embodiments, one or more color sensors **122,** for example and without limitation an RGB sensor can be provided in the collection reservoir **116.** In some embodiments, the one or more color sensors **122** can be used to detect and measure a color or shade of the urine. This information can be used to determine the health of the patient, for example, determining whether the patient is dehydrated and needs more fluids or electrolytes, or is bleeding, etc. In some embodiments, the TDS meter **120** and/or the one or more color sensors **122** can be in communication with the one or more processors via a wired or wireless connection and can transmit and receive data and instructions, respectively, regarding their functions and output captures.

FIG. 4B depicts the rear of an example collection reservoir **116,** each of the collection reservoirs **116** comprising a quick connect port **124** that allows it to quickly connect to the smart catheter **100.** The quick connect port **124** can be configured to allow fluid, including urine, to drain and collect into the collection reservoir **116** from the flexible tube **106** that connects to the top of the housing. Although not depicted in the figures, the smart catheter **100** of the present disclosure can comprise an infrastructure inside the housing that connects the flexible tube **106** to the quick connect port **124.** In some embodiments, the infrastructure inside the smart catheter **100** can comprise a switch or electric valve which can be configured to select which of the one or more collection reservoirs **116** the flexible tube **106** drains into. In some embodiments, the one or more processors is configured to operate the infrastructure, including the one or more switches or electric valves. In this case, either automatically or manually, the processor is configured to actuate the switch or electric valve to select which collection reservoir **116** is to receive urine. For example and without limitation, once the first collection reservoir **116A** gets full or to a certain level, the processor is configured to actuate the switch or valve such that urine or other fluid starts draining into the second collection reservoir **116B.** Additionally, if there is a malfunction in a collection reservoir **116,** or for some other reason, the smart catheter **100** is configured to allow medical staff to manually select a different collection reservoir **116** and the switch will be actuated to change to the other reservoir. According to the invention, if the collection reservoir **116** currently being drained into is removed from the device, the smart catheter **100** is configured to automatically move over to any of the other available collection reservoirs **116** that are not themselves already full or malfunctioning.

Additionally, one or more collection reservoirs **116** comprises an electrical connection **128** configured to not only help power the various sensors, meters, and other components within the one or more collection reservoir **116,** but also to connect the one or more processors to the various sensors, meters, and other components for performing the actions described herein. For example, and without limitation, each of the TDS meter **120** outputs and the one or more color sensors **122** can measure their respective characteristics of the urine or other fluid in the collection reservoir and then transmit those measurements to the one or more processors via the electrical connection **128.** This action can be performed automatically by the sensors themselves (i.e., without any request or query from the processor) or the one or more processors can query the sensors at a constant, periodic, or random time, or, a medical professional could manually request a check of the outputs of the sensors via the display **104** or other computing equipment that is in communication with the smart catheter **100.**

In some embodiments, the electrical connection **128** can be used by the smart catheter **100** to communicate with the collection reservoir **116** to determine a fluid level of the collection reservoir **116.** In some embodiments, the smart catheter **100** is configured to alarm when at least one of the collection reservoirs **116** is at least half-way full. Furthermore, in some embodiments, the smart catheter **100** is configured to alarm when there is no urine output or not sufficient urine output. These can be set parameters as well. In some embodiments, the alarm could be, for example and without limitation, a signal, message, text message, electronic message, electronic mail, or other appropriate message sent to medical professional mobile devices, tablets, computers, or mobile work stations so that they will be informed of the alarm. Any messages, alarms, or warnings sent via textual or electronic message can include such information as the type of warning, how much of the collection reservoir **116** is full, what its fill level is at, and any other relevant information such as the associated patient, hospital room, or other suitable identifier.

In some other embodiments, the alarm could be, for example a sound made by the smart catheter **116** via a speaker (not shown). In some embodiments, the smart catheter **100** is configured such that it can alarm using an automated voice such as "RESERVOIR FULL", "ALL RESERVOIRS FULL", "RESERVOIR HALF FULL", "RESERVOIR IS X% FULL" (X being any percentage), or any other programmable automated voice sound. In some embodiments, every time urine is deposited into one or more reservoirs, the smart catheter **100** can be configured to alert any of the voice alarms above. In some embodiments, the alarm could be a beep or other sound that occurs once or a few times just to alert the medical professionals working with the patient that the collection reservoir **116** is halfway full. In some embodiments, the smart catheter **100** is configured to trigger an alarm when at least one of the collection reservoirs **116** is almost full and there is only a selectively predetermined volume left, such as 100ml, before the collection reservoir **116** is completely full. In this particular case, the alarm indicating that the collection reservoir **116** is almost full can be a different sound or have some other indicia (e.g., different tune, different number of beeps, etc.) that indicates that it is a different alarm than the one indicating that the collection reservoir **116** is halfway full. In a similar fashion, if a predetermined minimum threshold of urine has not been collected by the smart catheter **100,** as described herein, then an alarm, like those described herein, can be triggered. In some embodiments, if the amount of urine that is collected by the smart catheter **100,** as described herein, does not fall within a certain predetermined threshold within a specific amount of time, then an alarm can be triggered, as described herein. In some embodiments, the system will notify of critical values or when set parameters are exceeded. These parameters can be patient specific and thus can be altered or customized by the healthcare officials via the display **104** or via transmissions to the one or more processors on board the smart catheter **100.**

In some embodiments, when only a single collection reservoir **116** is inserted in the smart catheter **100,** once it is completely full, a similar alarm to those described above will sound and/or appropriate electronic messages described above will be sent. In some embodiments, the smart catheter **100** is configured such that any alarm conveying a sound such as a beep or other alarm sound will continue to alarm until the collection reservoir **116** is exchanged or until the smart catheter **100** is turned off (e.g., if the patient is no longer hooked up to the smart catheter 100). Similarly, if the smart catheter **100** comprises more than one collection reservoir **116,** such as the two collection reservoirs **116A** and **116B** in FIG. 3A and FIG. 3B, the alarm that continuously sounds or alerts will do so when all of the collection reservoirs **116** are full. In some embodiments, these limits described hereinabove can be set and adjusted by medical staff monitoring the smart catheter **100** to address differences in patients and differences in the size of potential collection reservoirs **116.** For example and without limitation, the alarms can sound off at any particular set limit and more than three alarms can be set. For example and without limitation, a plurality of alarms can be set to trigger at various fill levels of the collection reservoir **116.** The lengths, sounds, chirps, messages, etc. can all be modified or changed to help medical staff to differentiate between which alarm goes to which fill level. All of the alarms, measurements, and other various features of the smart catheter **100** are customizable and adjustable and has sufficient memory and storage to be able to have a plurality of predetermined and preset limits and alarms.

Additionally, in some embodiments, when the smart catheter **100** detects a malfunction with the connection to one or more of the collection reservoirs **116** (or any of the sensors or meters therein), or a malfunction of the display **104,** lighting element **108,** or any other component of the smart catheter **100,** an alarm is configured to be triggered, similar to the alarms described above. In some embodiments, if possible the lighting element **108** can flash a different color if the malfunction has to do with the display **104** or if the malfunction is such that the display **104** cannot be powered or, if the display **104** can be powered, an error message indicative of the malfunction can be displayed on the display **104.**

As illustrated in FIG. 4B, in some embodiments, the collection reservoir **116** can comprise a groove to locate and hold the collection reservoir **116.** Although they are depicted herein as substantially cylindrical, those having ordinary skill in the art will appreciate that the collection reservoirs **116** and thus, the shape of the smart catheter **100** can be of any suitable shape and size. For example, the collection reservoirs **116** can be flask shaped, cylindrical, bag shaped, bowl shaped, cube shaped, shaped like a rectangular prism, or any suitable three-dimensional shape that can collect and hold fluid. The smart catheter **100** can be modified from the shape illustrated in the present figures to fit any shape described above or any three-dimensional shape.

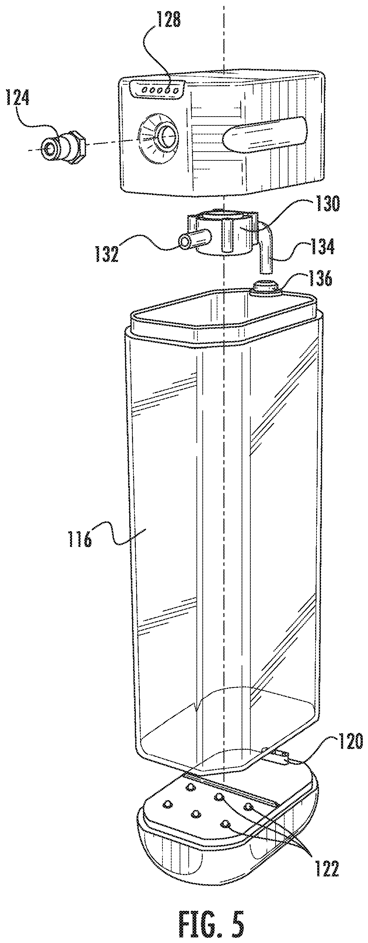

FIG. 5 illustrates an exploded view of an example collection reservoir **116** of the present disclosure. In some embodiments, the collection reservoir **116** comprises an inlet port **132** that connects to the infrastructure of the smart catheter **100** via the quick connect port **124.** In some embodiments, the inlet port **132** connects to the switch or electronic valve that determines or selects which collection reservoir **116** is meant to receive urine or other fluid at the current time. Once the collection reservoir **116** is properly inserted into the smart catheter **100** and the quick connect port **124** is connected to the infrastructure of the smart catheter **100** and the particular collection reservoir **116** is selected to receive urine, the urine flows through the inlet port **132** to the flow meter **130** which is configured to measure the amount of urine that is collected in the collection reservoir **116.** Once the urine flows through the flow meter **130,** it flows out the egress port **134** and into the spout **136** of the collection reservoir **116.** In some embodiments, the flow meter **130** is configured to measure the volume of urine that flows through it via any suitable means. For example and without limitation, in some embodiments, the flow meter **130** is configured to calculate the flow rate by determining the number of milliliters of fluid per second (or other suitable volume per time period) that flows into the collection reservoir **116** and multiply that by the number of seconds that it detects urine flowing at the given rate. In some embodiments, the flow meter **130** can use more or less accurate measurements of the flow rate to determine a more precise volume of urine in the collection reservoir **116.** In some embodiments, the flow meter **130** can be a part of the flexible tube **106** itself and not part of the collection reservoir **116.** In other words, the flow meter **130** can be positioned at any suitable location along the flexible tube **106.** Furthermore, the flow meter **130** can be integrated into the flexible tube **106** or be a separate piece as shown in FIG. 5. In such an instance where the flow meter **130** is positioned outside of the housing, the flow meter **130** is still in communication with and subject to instructions from the one or more processors via wireless or wired connection.

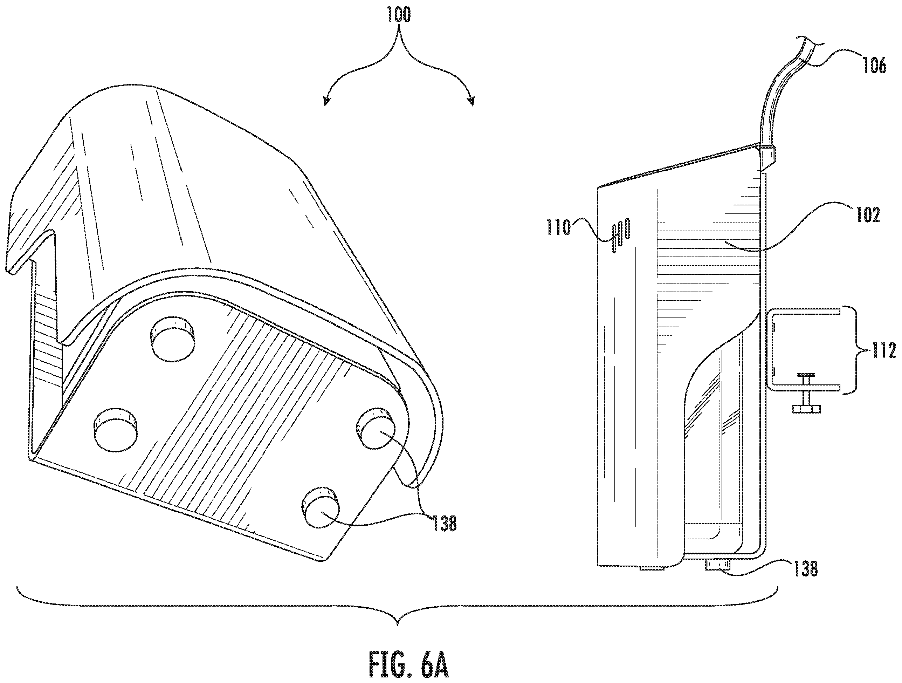

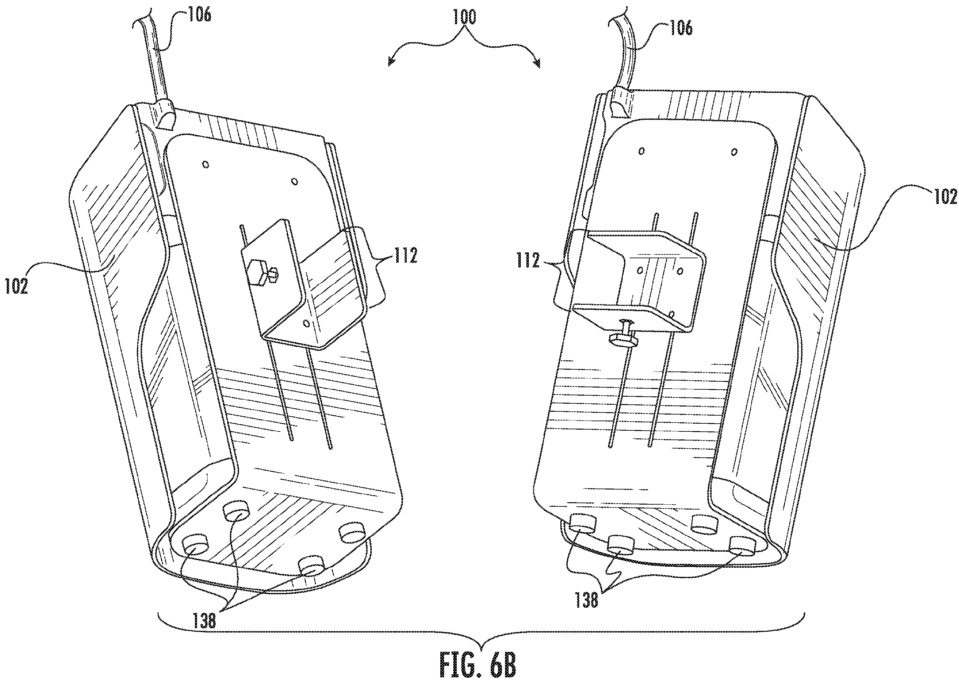

Referring to FIG. 6A and FIG. 6B, in some embodiments, the smart catheter **100** can comprise one or more rubber feet **138** on the bottom of the device such that if the device were positioned on a surface, any sliding of the device would be minimized. Additionally, as illustrated in FIG. 6B, in some embodiments, the clamp and/or bracket **112** on the back can be rotated such that the opening of the clamp and/or bracket **112** is either parallel to the length of the housing of the smart catheter **100** or perpendicular to the length of the housing of the smart catheter **100.** Those having ordinary skill in the art will appreciate that the clamp and/or bracket **112** can be adjusted up or down along a track on the back of the smart catheter **100** as well. Moreover, in some embodiments, the clamp and/or bracket **112** is rotatable in place and doesn't need to be removed in order to rotate it to be parallel with or perpendicular with respect to the smart catheter **100.** Furthermore, in some embodiments, the clamp and/or bracket **112** can be configured to mount the smart catheter **100** to a standard hospital bed frame, transport poles, or any other suitable location. For example and without limitation, the clamp or bracket **112** can be configured to attach the smart catheter **100** housing or enclosure to a bed or other frame, pole, or other suitable attachment point with dimensions of ½", 1", 1 ½", 2", 2 ½", etc.

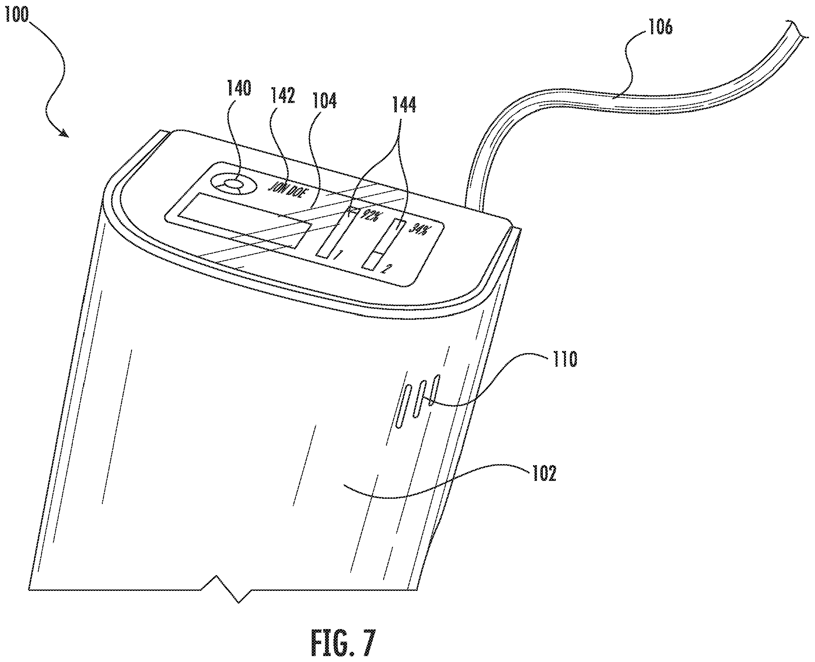

Referring to FIG. 7, in some embodiments, the smart catheter **100** of the present disclosure is configured to present various pieces of information on the display **104.** For example and without limitation, the display **104** can be configured to show information identifying the patient for which the particular smart catheter **100** is being used for. Such identifying information could be a picture **140** or avatar of the patient or the patient's name **142,** or patient identification number. In addition, in some embodiments, fluid levels **144** or capacities of each collection reservoir **116** can be displayed. In some embodiments, the one or more processors situated within the housing of the smart catheter **100** can control what and when items are displayed. In some embodiments, the alarms described herein can coincide with various indicators flashing on the display **104** to help alert healthcare workers of the issue. Additionally, in some embodiments, the display can flash the fluid level **144** indicator of one more of the collection reservoirs **116** when they have reached certain thresholds. In this way, medical staff can easily determine which collection reservoir **116** is full before even opening up the door **102.** In some embodiments, the display **104** can also show pertinent information about the urine, such as for example, any dissolved solids statistics or other information. In some embodiments, the display **104** is configured to light up to match the color of the urine. In some further embodiments, the display **104** has the ability to have sensor motion to turn the light on from dim to bright.

Those having ordinary skill in the art will appreciate that the display **104** can be configured to display any appropriate information that relates to the patient, medical workers, the status of the smart catheter **100** or any of its parts, etc. To allow a medical professional who is operating and monitoring the smart catheter **100** to have easier control over the device, the display **104** can be a touchscreen display that has multiple different pages, folders, and buttons that can be displayed, changed, altered, customized, etc. Additionally, in some embodiments, the one or more processors can be in communication with one or more external devices. In some embodiments, the external devices comprises servers or other computers hosting the patient's medical records and/or medical chart. In such an embodiment, the one or more processors can be configured to automatically measure the amount or volume of urine according to predetermined sets of time (e.g., continuously, periodically, randomly, every 30 minutes or Q one hour, etc.) in one or more of the collection reservoirs **116** and transmit a volume of urine in total or in each of the collection reservoirs **116.** Additionally, information about the urine gleaned from the various sensors can also be recorded and sent to the medical records and/or medical chart for updating.

Moreover, in some embodiments, the external device comprises a tablet, computer, mobile device, phone, smart watch, audio device, handheld documentation device or display device. In some embodiments such a catheter as provided herein further comprises a power source, a computer, memory, a receiver or transmitter, an accelerometer, a speaker, microphone, or a tactile signal device, wherein the power source, computer, memory, receiver or transmitter, accelerometer, speaker or tactile signal device are interconnected with one another. In some embodiments such a catheter as provided herein further comprises a computer program product comprising computer executable instructions embodied in a computer readable medium for performing steps comprising receiving an electrical signal from a measuring apparatus, processing the electrical signal to calculate data pertaining to a measured volume, and relaying the data to the electronic display, speaker, tactile signal device or external device. In some embodiments, a wireless receiver is configured to receive data wirelessly and transfer it to a computer, wherein the computer is configured to process the data and transmit it to the display, speaker or tactile signal device.

The functions and subject matter described herein, especially with respect to the one or more processors described herein, can in some embodiments be implemented using a computer program product comprising computer executable instructions embodied in a computer readable medium. Such computer readable medium can be stored in memory and implemented by computer. Exemplary computer readable media suitable for implementing the subject matter described herein include disk memory devices, chip memory devices, application specific integrated circuits, programmable logic devices, and downloadable electrical signals. In addition, a computer program product that implements the subject matter described herein may be located on a single device or computing platform or may be distributed across multiple devices or computing platforms.

All of the above measurements and transmissions can be done in real time or after the fact (i.e., as soon as urine flows and stops flowing into the collection reservoir **116,** the smart catheter **100** can transmit the volume data and urine characteristics data to the medical records server). In some embodiments, the smart catheter **100** can comprise a transmitter, transmission device, or transceiver for making a wired or wireless connection to the medical records and/or medical chart server/computer. Moreover, in some embodiments, the smart catheter **100** comprises a receiver, such as, for example and without limitation, a wireless or wired receiver configured to receive data from an external device. Using the one or more processors and transmitter and/or receiver, any component of the smart catheter **100** can receive or transmit data to/from any suitable external or internal device (i.e., such as the medical records or medical chart server, mobile phones, tablets, medical equipment, etc.). A wireless receiver and/or transmitter can be configured to wirelessly receive and/or transmit data and information via wireless signal. By way of example and not limitation, such wireless forms of communication can comprise Wi-Fi and Bluetooth. As described herein, with integrated wireless communication capabilities, catheters can exchange information and/or data, i.e. receive and/or transmit, with another device, such as but not limited to a tablet, computer, phone, smart watch, audio device or display device.

In some embodiments, the smart catheter **100** can be powered by a wired power cable connected to an electrical outlet. In further embodiments, the smart catheter **100** can be powered by a battery or other suitable power source. In either embodiments, the smart catheter **100** can be configured to trigger and sound an alarm as described herein when either the power connection is inadequate, or there is a malfunction with the power connector or the battery or other power source. Additionally, in some embodiments, the smart catheter **100** can be operable using speech recognition. In support of this feature, some embodiments of the smart catheter **100** of the present disclosure comprise speakers and an audio input device, such as a microphone, that allow a user to speak to the smart catheter **100** to issue commands or requests. Moreover, in such an embodiment, the one or more processors of the smart catheter **100** can be configured to operate an artificial intelligence program that is configured to receive spoken commands and respond with additional audio feedback or perform tasks in connection with the commands. In this way, any of the parameters, actions, services, or performances that can be performed automatically, or manually be a person touching the smart catheter **100** can also be performed via voice command using speech recognition.

### Smart Chest Tube

In some embodiments of the present disclosure, the smart catheter **100** can be retrofitted to act as a smart chest tube, instead. Provided herein are smart chest tubes, also referred to herein as precise chest tubes, KG chest tubes, chest tubes and/or electronic chest tubes. A chest tube is a flexible plastic tube that is inserted through the chest wall and into the pleural space or mediastinum. It is used to remove air, fluid, pleural effusion, blood, chyle, or pus from the intrathoracic space. It is also known as a Bülau drain or an intercostal catheter.

In some cases, pressure around the lungs is lower than atmospheric pressure outside the body. The aims for an adequate chest drainage system to be fulfilled are: (i) remove fluid and air as promptly as possible; (ii) prevent drained air and fluid from returning to the pleural space, restore negative pressure in the pleural space to re-expand the lung. Thus, in some embodiments, a drainage device can: (i) allow air and fluid to leave the chest; (ii) contain a one-way valve to prevent air and fluid from returning to the chest; (iii) comprise a design so that the device is below the level of the chest tube for gravity drainage. An underwater seal chest drainage system can be used to restore proper air pressure to the lungs, re-inflate a collapsed lung as well as remove blood and other fluids. The system is a two-chambered or three-chambered plastic unit with vertical columns bringing measurements marked in milliliters. The thoracic drainage devices cover a wide range and have evolved considerably since their introduction. The basic design principle of these systems has been the avoidance of air entrance in the pleural cavity during the various phases of the respiratory cycle and continuous drainage of air and fluid from the pleural cavity. The water seal chamber, which is connected in series to the collection chamber, allows air to pass down through a straw or narrow channel and bubble out through the bottom of the water seal. Since air must not return to the patient, a water seal is considered one of the safest and cost-effective means for protecting the patient, in addition to being a very useful diagnostic tool. The water seal column is calibrated and acts as a water manometer for measuring intrathoracic pressure.

In a traditional water seal operating system, fluids drain from the patient directly into a large collection chamber via a tube, e.g. a six-foot 3/8-inch tube. As drainage fluids collect in this chamber, a nurse or other practitioner can record the amount of fluid that collects on a specified schedule. Unfortunately, the measuring and recording of the drainage fluid is still done manually. What is needed is an automated and accurate measurement and recording device.

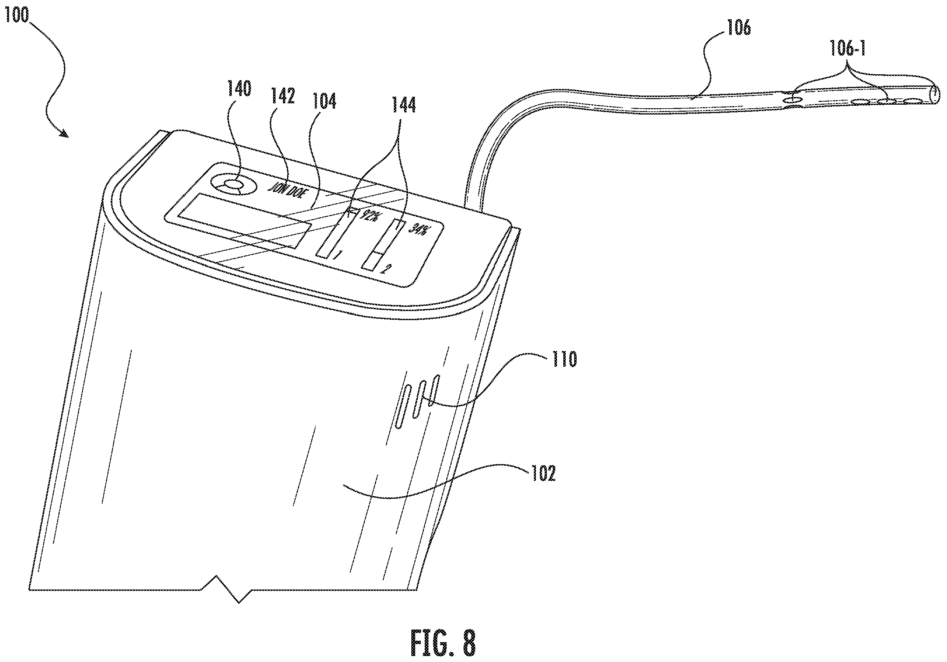

Referring to FIG. 8, in some embodiments, the smart chest tube of the present disclosure can look virtually identical to the smart catheter **100** of FIG. 1 - FIG. 7 and have virtually all the same components. However, for a smart chest tube, additional components are required, as those having ordinary skill in the art will fully appreciate. These components are well known in the art. Specifically, the measuring devices, sensors, and other pieces of equipment described hereinabove can be altered to be able to receive and measure air, fluid, pleural effusion, blood, chyle, or pus flowing from the intrathoracic space through the chest tube. The flexible tube **106** can be replaced or altered to operate as a chest tube, wherein the flexible tube **106** comprises one or more openings at one end **106-1,** and a drainage port at the opposing end. In some embodiments, the one or more openings **106-1** can be located at the end face of the flexible tube **106,** or they can be axially aligned, meaning they run down the length of the flexible tube **106,** starting near the end. In some other embodiments, the one or more openings **106-1** can be circumferentially aligned, meaning around the circumference of the flexible tube **106.**

In some embodiments, the flow meter **130** of FIG. 5 is configured to measure an amount of air, fluid, pleural effusion, blood, chyle, and/or pus flowing therethrough, instead of urine. The flow meter **130** can be adapted to properly measure almost any fluid flowing therethrough.

The measured volume of air, fluid, pleural effusion, blood, chyle, or pus determined by the flow meter **130** can be transmitted as data to an external device, e.g. a computer or receiver. As described herein, the smart chest tube device can in some embodiments comprise a transmitter or transmission device to wirelessly transmit the data to an external device. The smart chest tube can in some embodiments also comprise a receiver configured to receive data from an external device.

In some embodiments, when the device is retrofitted as a smart chest tube, the device is configured to measure and calculate the volume of air, fluid, pleural effusion, blood, chyle, or pus produced by a patient and activate an alarm if such volume output exceeds or does not meet a set or predetermined threshold, or as described above, if the collection reservoirs **116** are full, about to be full, or if there is a malfunction. All of the features and aspects described above with respect to the smart catheter **100** can be retrofitted and slightly altered to account for any differences in the operations of a urinary catheter versus a chest tube. For example, tubes and ports sizes can be increased and materials can be altered to make it more available for puss and chyle storage.

Additionally, for the smart chest tube application, the smart catheter 100 as described herein can be retrofitted with a suction control chamber to help suction the fluid being drained from the chest cavity of the patient.

The present subject matter can be embodied in other forms without departure from the spirit and essential characteristics thereof. The embodiments described therefore are to be considered in all respects as illustrative and not restrictive. Although the present subject matter has been described in terms of certain specific embodiments, other embodiments that are apparent to those of ordinary skill in the art are also within the scope of the present subject matter.

## Claims

1. An automated catheter (100) comprising:
a flexible tube (106) with an opening (106-1) at a first end;
a drainage port at an opposing second end;
an inflatable balloon (106-2) at the first end, and proximate to the opening (106-1);
a balloon port at the second end and proximate to the drainage port;
a measuring device, comprising one or more sensors, integrated into or attached to the flexible tube (106) and configured to measure an amount of fluid flowing through the flexible tube; and
at least two collection reservoirs (116), wherein the drainage port is configured to connect or otherwise attach to the at least two collection reservoirs (116).
wherein the automated catheter (100) is configured to drain urine from a bladder of a patient and is configured to deposit the urine in the at least two collection reservoirs (116);
wherein the measuring device is a flow meter sensor, wherein the flow meter sensor is configured to measure the amount of urine deposited into the at least two collection reservoirs (116);
wherein, when a first collection reservoir (116) of the at least two collection reservoirs (116) is removed from the automated catheter (100), the automated catheter (100) is configured to automatically select a second collection reservoir (116) of the at least two collection reservoirs (116) and start draining urine into the second collection reservoir (116).

2. The automated catheter (100) of claim 1, wherein the automated catheter (100) comprises one or more processors, one or more transmitters, and one or more receivers;
wherein the one or more processors is configured to transmit and receive data, via the one or more transmitters or one or more receivers, to or from one or more computing device in communication with the automated catheter (100).

3. The automated catheter (100) of claim 2, wherein the transmitted data comprises urine flow data;
wherein at least one of the one or more computing devices is configured to store and monitor the urine flow data;
wherein the one or more processors and the at least one computing device is configured to monitor the amount of urine deposited into the at least two collection reservoirs (116) of the automated catheter (100); and
wherein the one or more processors is configured to transmit a warning signal or trigger an alarm when at least one of the at least two collection reservoirs (116) of the automated catheter (100) reaches one or more thresholds.

4. The automated catheter (100) of claim 1, wherein components of the automated catheter (100) are housed in an enclosure comprising a display (104) configured to display fill levels of the at least two collection reservoirs (116) of the automated catheter (100);
wherein the enclosure further comprises a transparent door (102), and
wherein each of the at least two collection reservoirs (116) is transparent.

5. The automated catheter (100) of any of the previous claims, wherein one or more collection reservoir (116) of the at least two collection reservoirs (116) comprises a total dissolved solids meter and/or a color sensor configured to detect and measure a color or shade of a fluid, and wherein the at least two collection reservoirs (116) comprise a quick connect port to connect the at least two collection reservoirs (116) to the automated catheter (100).

6. An automated catheter system comprising:
an automated catheter (100) of any of the above claims; and
an external device, wherein the external device comprises a tablet, computer, phone, smart watch, audio device or display device.

7. The automated catheter system of claim 6, wherein the system further comprises:
a power source, one or more processors, memory, a receiver or transmitter, a display, an accelerometer, and a speaker and/or a tactile signal device,
wherein the automated catheter (100), the power source, the one or more processors, the memory, the receiver or transmitter, the display, the accelerometer, and the speaker and/or the tactile signal device are interconnected with one another.

8. The automated catheter system of claim 6 or 7, wherein the display is configured to display information about the patient and/or the automated catheter,
wherein the display is configured to display identification information about the patient as well as information regarding the patient's urine; and
wherein the display is configured to display information about a capacity of each of the at least two collection reservoirs (116) or a warning or error message.

9. The automated catheter system of any of claims 6 to 8, comprising a computer program product comprising computer executable instructions embodied in a computer readable medium for performing steps comprising receiving an electrical signal from a measuring apparatus, processing the electrical signal to calculate data pertaining to a measured volume, and relaying the data to the electronic display, speaker, tactile signal device or external device.

10. The automated catheter system of any of claims 6 to 9, wherein the automated catheter system is configured to measure and calculate a volume of urine or other body fluid produced by a patient every 30 minutes or one hour, wherein the calculated volume is processed by a computer and entered into an electronic charting system.

11. The automated catheter system of any of claims 6 to 10, wherein the automated catheter system is configured to measure and calculate the volume of urine or other body fluid produced by a patient and activate an alarm if the volume of urine and/or other body fluid is above and/or below a predetermined threshold.

12. The automated catheter (100) of any of claims 1 to 5, wherein each of the at least two collection reservoirs (116) comprises an electrical connection (128) configured to provide power to various components in a respective collection reservoir (116) and to connect the one or more processors to the various components in the respective collection reservoir (116).

## Patentansprüche

1. Automatisierter Katheter (100), umfassend:
einen flexiblen Schlauch (106) mit einer Öffnung (106-1) an einem ersten Ende;
einen Entwässerungsanschluss an einem gegenüberliegenden zweiten Ende;
einen aufblasbaren Ballon (106-2) an dem ersten Ende und nahe der Öffnung (106-1);
einen Ballonanschluss an dem zweiten Ende und nahe dem Entwässerungsanschluss;
eine Messvorrichtung, die einen oder mehrere Sensoren umfasst, in den flexiblen Schlauch (106) integriert oder an diesem befestigt ist und konfiguriert ist, eine Menge an Fluid zu messen, die durch den flexiblen Schlauch strömt; und
mindestens zwei Sammelbehälter (116), wobei der Entwässerungsanschluss konfiguriert ist, um mit den mindestens zwei Sammelbehältern (116) verbunden oder anderweitig daran befestigt zu werden,
wobei der automatisierte Katheter (100) konfiguriert ist, um Urin aus einer Blase eines Patienten abzuleiten, und konfiguriert ist, um den Urin in die mindestens zwei Sammelbehälter (116) abzugeben;
wobei die Messvorrichtung ein Durchflussmessersensor ist, wobei der Durchflussmessersensor konfiguriert ist, um die Menge an Urin zu messen, die in die mindestens zwei Sammelbehälter (116) abgegeben wird;
wobei, wenn ein erster Sammelbehälter (116) der mindestens zwei Sammelbehälter (116) von dem automatisierten Katheter (100) entfernt wird, der automatisierte Katheter (100) konfiguriert ist, um automatisch einen zweiten Sammelbehälter (116) der mindestens zwei Sammelbehälter (116) auszuwählen und mit dem Ableiten von Urin in den zweiten Sammelbehälter (116) zu beginnen.

2. Automatisierter Katheter (100) nach Anspruch 1, wobei der automatisierte Katheter (100) einen oder mehrere Prozessoren, einen oder mehrere Sender und einen oder mehrere Empfänger umfasst;
wobei der eine oder die mehreren Prozessoren konfiguriert sind, um Daten über den einen oder die mehreren Sender oder den einen oder die mehreren Empfänger an eine oder von einer oder mehreren Rechenvorrichtungen in Kommunikation mit dem automatisierten Katheter (100) zu senden und zu empfangen.

3. Automatisierter Katheter (100) nach Anspruch 2, wobei die übertragenen Daten Urinflussdaten umfassen;
wobei mindestens eine der einen oder mehreren Rechenvorrichtungen konfiguriert ist, um die Urinflussdaten zu speichern und zu überwachen;
wobei der eine oder die mehreren Prozessoren und die mindestens eine Rechenvorrichtung konfiguriert sind, um die Menge an Urin zu überwachen, die in die mindestens zwei Sammelbehälter (116) des automatisierten Katheters (100) abgegeben wird; und
wobei der eine oder die mehreren Prozessoren konfiguriert sind, um ein Warnsignal zu übertragen oder einen Alarm auszulösen, wenn mindestens einer der mindestens zwei Sammelbehälter (116) des automatisierten Katheters (100) einen oder mehrere Schwellenwerte erreicht.

4. Automatisierte Katheter (100) nach Anspruch 1, wobei Komponenten des automatisierten Katheters (100) in einem Gehäuse untergebracht sind, das eine Anzeige (104) umfasst, die konfiguriert ist, um Füllstände der mindestens zwei Sammelbehälter (116) des automatisierten Katheters (100) anzuzeigen;
wobei das Gehäuse ferner eine transparente Tür (102) umfasst, und wobei jeder der mindestens zwei Sammelbehälter (116) transparent ist.

5. Automatisierter Katheter (100) nach einem der vorstehenden Ansprüche, wobei ein oder mehrere Sammelbehälter (116) der mindestens zwei Sammelreservoirs (116) ein Messgerät für gelöste Gesamtfeststoffe und/oder einen Farbsensor umfasst, der konfiguriert ist, um eine Farbe oder einen Farbton eines Fluids zu erfassen und zu messen, und wobei die mindestens zwei Sammelbehälter (116) einen Schnellverbindungsanschluss umfassen, um die mindestens zwei Sammelbehälter (116) mit dem automatisierten Katheter (100) zu verbinden.

6. Automatisiertes Kathetersystem, umfassend:
einen automatisierten Katheter (100) nach einem der vorstehenden Ansprüche; und
eine externe Vorrichtung, wobei die externe Vorrichtung ein Tablet, einen Computer, ein Telefon, eine Smartwatch, eine Audiovorrichtung oder eine Anzeigevorrichtung umfasst.

7. Automatisierter Kathetersystem nach Anspruch 6, wobei das System ferner Folgendes umfasst:
eine Energiequelle, einen oder mehrere Prozessoren, einen Speicher, einen Empfänger oder Sender, eine Anzeige, einen Beschleunigungsmesser und einen Lautsprecher und/oder eine taktile Signalvorrichtung,
wobei der automatisierte Katheter (100), die Energiequelle, der eine oder die mehreren Prozessoren, der Speicher, der Empfänger oder Sender, die Anzeige, der Beschleunigungssensor und der Lautsprecher und/oder die taktile Signalvorrichtung miteinander verbunden sind.

8. Automatisiertes Kathetersystem nach Anspruch 6 oder 7, wobei die Anzeige konfiguriert ist, um Informationen über den Patienten und/oder den automatisierten Katheter anzuzeigen, wobei die Anzeige konfiguriert ist, um Identifikationsinformationen über den Patienten sowie Informationen bezüglich des Urins des Patienten anzuzeigen; und
wobei die Anzeige konfiguriert ist, um Informationen über eine Kapazität jedes der mindestens zwei Sammelbehälter (116) oder eine Warn- oder Fehlermeldung anzuzeigen.

9. Automatisiertes Kathetersystem nach einem der Ansprüche 6 bis 8, umfassend ein Computerprogrammprodukt, das in einem computerlesbaren Medium verkörperte computerausführbare Anweisungen umfasst, zum Ausführen von Schritten, umfassend das Empfangen eines elektrischen Signals von einer Messvorrichtung, das Verarbeiten des elektrischen Signals zum Berechnen von Daten bezüglich eines gemessenen Volumens und das Weiterleiten der Daten an die elektronische Anzeige, den Lautsprecher, die taktile Signalvorrichtung oder die externe Vorrichtung.

10. Automatisiertes Kathetersystem nach einem der Ansprüche 6 bis 9, wobei das automatisierte Kathetersystem konfiguriert ist, um ein Volumen von Urin oder einer anderen Körperflüssigkeit, das von einem Patienten produziert wird, alle 30 Minuten oder eine Stunde zu messen und zu berechnen, wobei das berechnete Volumen von einem Computer verarbeitet und in ein elektronisches Aufzeichnungssystem eingegeben wird.

11. Automatisiertes Kathetersystem nach einem der Ansprüche 6 bis 10, wobei das automatisierte Kathetersystem konfiguriert ist, um das Volumen von Urin oder anderer Körperflüssigkeit, die von einem Patienten produziert wird, zu messen und zu berechnen und einen Alarm zu aktivieren, wenn das Volumen von Urin und/oder anderer Körperflüssigkeit über und/oder unter einem vorbestimmten Schwellenwert liegt.

12. Automatisierter Katheter (100) nach einem der Ansprüche 1 bis 5, wobei jedes der mindestens zwei Sammelbehälter (116) eine elektrische Verbindung (128) umfasst, die konfiguriert ist, um verschiedene Komponenten in einem jeweiligen Sammelbehälter (116) mit Strom zu versorgen und den einen oder die mehreren Prozessoren mit den verschiedenen Komponenten in dem jeweiligen Sammelbehälter (116) zu verbinden.

## Revendications

1. Cathéter automatisé (100) comprenant :
un tube flexible (106) avec une ouverture (106-1) à une première extrémité ;
un orifice de drainage à une seconde extrémité opposée ;
un ballonnet gonflable (106-2) à la première extrémité, et à proximité de l'ouverture (106-1) ;
un orifice de ballonnet à la seconde extrémité et à proximité de l'orifice de drainage ;
un dispositif de mesure, comprenant un ou plusieurs capteurs, intégré dans ou fixé au tube flexible (106) et configuré pour mesurer une quantité de fluide s'écoulant à travers le tube flexible ; et
au moins deux réservoirs de collecte (116), dans lequel l'orifice de drainage est conçu pour être relié ou autrement fixé aux au moins deux réservoirs de collecte (116),
dans lequel le cathéter automatisé (100) est conçu pour drainer l'urine d'une vessie d'un patient et est conçu pour déposer l'urine dans les au moins deux réservoirs de collecte (116) ;
dans lequel le dispositif de mesure est un capteur de débitmètre, dans lequel le capteur de débitmètre est configuré pour mesurer la quantité d'urine déposée dans les au moins deux réservoirs de collecte (116) ;
dans lequel, lorsqu'un premier réservoir de collecte (116) des au moins deux réservoirs de collecte (116) est retiré du cathéter automatisé (100), le cathéter automatisé (100) est conçu pour sélectionner automatiquement un second réservoir de collecte (116) des au moins deux réservoirs de collecte (116) et commencer à drainer l'urine dans le second réservoir de collecte (116).

2. Cathéter automatisé (100) selon la revendication 1, dans lequel le cathéter automatisé (100) comprend un ou plusieurs processeurs, un ou plusieurs émetteurs et un ou plusieurs récepteurs ;
dans lequel le ou les processeurs sont configurés pour transmettre et recevoir des données, par l'intermédiaire du ou des émetteurs ou du ou des récepteurs, vers ou depuis un ou plusieurs dispositifs informatiques en communication avec le cathéter automatisé (100).

3. Cathéter automatisé (100) selon la revendication 2, dans lequel les données transmises comprennent des données de débit urinaire ;
dans lequel au moins l'un du ou des dispositifs informatiques est configuré pour stocker et surveiller les données de débit urinaire ;
dans lequel le ou les processeurs et l'au moins un dispositif informatique sont configurés pour surveiller la quantité d'urine déposée dans les au moins deux réservoirs de collecte (116) du cathéter automatisé (100) ; et
dans lequel le ou les processeurs sont configurés pour transmettre un signal d'avertissement ou déclencher une alarme lorsqu'au moins l'un des au moins deux réservoirs de collecte (116) du cathéter automatisé (100) atteint un ou plusieurs seuils.

4. Cathéter automatisé (100) selon la revendication 1, dans lequel des composants du cathéter automatisé (100) sont logés dans un boîtier comprenant un dispositif d'affichage (104) configuré pour afficher les niveaux de remplissage des au moins deux réservoirs de collecte (116) du cathéter automatisé (100) ;
dans lequel l'enceinte comprend en outre une porte transparente (102), et dans lequel chacun des au moins deux réservoirs de collecte (116) est transparent.

5. Cathéter automatisé (100) selon l'une quelconque des revendications précédentes, dans lequel un ou plusieurs réservoirs de collecte (116) des au moins deux réservoirs de collecte (116) comprennent un appareil de mesure des solides dissous totaux et/ou un capteur de couleur configuré pour détecter et mesurer une couleur ou une nuance d'un fluide, et dans lequel les au moins deux réservoirs de collecte (116) comprennent un orifice de liaison rapide pour relier les au moins deux réservoirs de collecte (116) au cathéter automatisé (100).

6. Système de cathéter automatisé comprenant :
un cathéter automatisé (100) selon l'une quelconque des revendications précédentes ; et
un dispositif externe, dans lequel le dispositif externe comprend une tablette, un ordinateur, un téléphone, une montre connectée, un dispositif audio ou un dispositif d'affichage.

7. Système de cathéter automatisé selon la revendication 6, dans lequel le système comprend en outre :
une source d'alimentation, un ou plusieurs processeurs, une mémoire, un récepteur ou un émetteur, un dispositif d'affichage, un accéléromètre, et un haut-parleur et/ou un dispositif de signal tactile,
dans lequel le cathéter automatisé (100), la source d'alimentation, le ou les processeurs, la mémoire, le récepteur ou l'émetteur, l'écran, l'accéléromètre, et le haut-parleur et/ou le dispositif de signal tactile sont interconnectés entre eux.

8. Système de cathéter automatisé selon la revendication 6 ou 7, dans lequel le dispositif d'affichage est configuré pour afficher des informations concernant le patient et/ou le cathéter automatisé, dans lequel le dispositif d'affichage est configuré pour afficher des informations d'identification concernant le patient ainsi que des informations concernant l'urine du patient ; et
dans lequel le dispositif d'affichage est configuré pour afficher des informations concernant une capacité de chacun des au moins deux réservoirs de collecte (116) ou un message d'avertissement ou d'erreur.

9. Système de cathéter automatisé selon l'une quelconque des revendications 6 à 8, comprenant un produit programme d'ordinateur comprenant des instructions exécutables par ordinateur incorporées dans un support lisible par ordinateur pour effectuer des étapes comprenant la réception d'un signal électrique provenant d'un appareil de mesure, le traitement du signal électrique pour calculer des données se rapportant à un volume mesuré, et la transmission des données à l'affichage électronique, au haut-parleur, au dispositif de signal tactile ou au dispositif externe.

10. Système de cathéter automatisé selon l'une quelconque des revendications 6 à 9, dans lequel le système de cathéter automatisé est configuré pour mesurer et calculer un volume d'urine ou d'un autre fluide corporel produit par un patient toutes les 30 minutes ou une heure, dans lequel le volume calculé est traité par un ordinateur et saisi dans un système de dossier électronique.

11. Système de cathéter automatisé selon l'une quelconque des revendications 6 à 10, dans lequel le système de cathéter automatisé est configuré pour mesurer et calculer le volume d'urine ou d'autre fluide corporel produit par un patient et activer une alarme si le volume d'urine et/ou d'autre fluide corporel est supérieur et/ou inférieur à un seuil prédéterminé.

12. Cathéter automatisé (100) selon l'une quelconque des revendications 1 à 5, dans lequel chacun des au moins deux réservoirs de collecte (116) comprend une connexion électrique (128) configurée pour fournir de l'énergie à divers composants dans un réservoir de collecte (116) respectif et pour connecter le ou les processeurs aux divers composants dans le réservoir de collecte (116) respectif.
